# EUROPEAN PATENT SPECIFICATION

(11) **EP 4 358 962 B1**
(45) Date of publication and mention of the grant of the patent: **04.02.2026**
(21) Application number: 22747090.3
(22) Date of filing: 23.06.2022
(51) Int. Cl.: A61K 31/485, A61K 31/05, A61K 31/4045, A61K 31/593, A61K 45/06, A61P 37/00

(54) **TREATMENT FOR AUTOIMMUNE DISEASES COMPRISING NALTREXONE OR A METABOLITE OR ANALOG THEREOF, A CANNABINOID AND A 5-HT RECEPTOR AGONIST**
BEHANDLUNG VON AUTOIMMUNKRANKHEITEN ENTHALTEND NALTREXON ODER EINEN METABOLIT ODER EIN ANALOG DESSELBEN, EIN CANNABINOID UND EINEN 5-HT-REZEPTOR-AGONIST
TRAITEMENT POUR MALADIES AUTO-IMMUNES COMPRENANT DE LA NALTREXONE OU UN MÉTABOLITE OU UN ANALOGUE DE CELLE-CI, UN CANNABINOÏDE ET UN AGONISTE DU RÉCEPTEUR 5-HT

(30) Priority: 23.06.2021 GB 202109021
(43) Date of publication of application: 01.05.2024
(73) Proprietor: LDN Pharma Limited, London EC2V 7BG (GB)
(72) Inventor: LIU, Wai, London EC2V 7BG (GB); THOMPSON, Ian, London EC2V 7BG (GB); HOOD, Francis, London EC2V 7BG (GB)
(74) Representative: Gill Jennings & Every LLP
(86) International application number: PCT/GB2022/051609
(87) International publication number: WO 2022/269267

(56) References cited:
- WO-A1-2016/064987
- WO-A1-2019/186207
- WO-A1-2020/188255
- BONAM SRINIVASA REDDY ET AL: "Parkinson's disease is an autoimmune disease: A reappraisal", AUTOIMMUNITY REVIEWS, ELSEVIER, AMSTERDAM, NL, vol. 19, no. 12, 22 October 2020 (2020-10-22), XP086371814, ISSN: 1568-9972, [retrieved on 20201022], DOI: 10.1016/J.AUTREV.2020.102684
- GON�ALVES ELAINE D ET AL: "Cannabinoid receptors as therapeutic targets for autoimmune diseases: where do we stand?", DRUG DISCOVERY TODAY, ELSEVIER, AMSTERDAM, NL, vol. 24, no. 9, 31 May 2019 (2019-05-31), pages 1845 - 1853, XP085824885, ISSN: 1359-6446, [retrieved on 20190531], DOI: 10.1016/J.DRUDIS.2019.05.023
- SANTIAGO MARINA ET AL: "Absence of entourage: Terpenoids commonly found in Cannabis sativa do not modulate the functional activity of [Delta]9-THC at human CB1and CB2 receptors", BIORXIV, 6 March 2019 (2019-03-06), pages 1 - 30, XP093235350, Retrieved from the Internet <URL:https://www.biorxiv.org/content/10.1101/569079v1.full.pdf> DOI: 10.1101/569079
- RENGASAMY KANNAN R R ET AL: "The role of flavonoids in autoimmune diseases: Therapeutic updates", PHARMACOLOGY & THERAPEUTICS, ELSEVIER, GB, vol. 194, 28 September 2018 (2018-09-28), pages 107 - 131, XP085583248, ISSN: 0163-7258, DOI: 10.1016/J.PHARMTHERA.2018.09.009
- THOMPSON CAITLIN ET AL: "Psychedelics as a novel approach to treating autoimmune conditions", IMMUNOLOGY LETTERS, vol. 228, 1 December 2020 (2020-12-01), NL, pages 45 - 54, XP055963447, ISSN: 0165-2478, Retrieved from the Internet <URL:https://www.sciencedirect.com/science/article/pii/S0165247820303977?via%3Dihub> DOI: 10.1016/j.imlet.2020.10.001

## Description

### Field of the invention

The invention relates to regimes of drug administration and drug combinations for use in the treatment of autoimmune diseases, as defined in the claims.

### Background to the Invention

Cannabinoids are a class of phenolic compounds abundantly produced in plants of the *Cannabis* genus. Various cannabinoid compounds have long been known to exhibit psychotropic effects in humans and are widely used recreationally, despite such use being illegal in many jurisdictions.

However, recent research has shown that certain cannabinoids are of therapeutic value against a diverse array of pathologies such as inflammatory disorders, neurodegenerative and psychiatric disorders, chronic pain, anxiety and PTSD. While cannabinoids are currently used to combat the wasting, emesis and nausea associated with cancer treatment, evidence exists which suggests that certain cannabinoid compounds may be effective in treating the underlying pathologies of cancers. Presently available evidence suggests that they do this through disruption of cancer cell migration, adhesion, and tumour vascularisation.

An endogenous cannabinoid-mediated signalling system functions in mammals, and on the balance of evidence probably in most other vertebrates too. In humans, two cannabinoid receptors have been identified and named CB1 and CB2, both of which are part of the G protein-coupled receptor superfamily.

Pharmacological evidence to date suggests that activation of the CB1 receptor is responsible for the majority of the psychotropic effects of cannabinoid intake, while therapeutic effects are mostly mediated by the CB2 receptor. As such, ideal therapeutic molecules will activate CB2 receptors in preference to CB1 receptors, alleviating unwanted psychotropic side effects. Cannabidiol (CBD), a component of *Cannabis* extract, is a cannabinoid with this binding profile which has recently generated considerable interest, but whose full mechanism of action is still being elucidated. In general however, natural cannabinoids do not tend to show high specificity for one or other receptor, but newly developed synthetic cannabinoids are available which bind more specifically.

It is sometimes the case that receptors in one signalling pathway, responsive to a particular ligand, can have their expression levels or downstream effects altered by a change in the signalling output of another seemingly separate signalling pathway. Such cross-modulation has been demonstrated between, for example, the signalling pathways following activation of the Growth Hormone and insulin receptors.

Autoimmune diseases are a broad spectrum of disorders characterised by the abnormal recognition and reaction to self-antigens by the immune system. Pathologically, autoimmune diseases can be caused by genetic factors, environmental factors, infectious agents or a combination of any number of the above. Different autoimmune diseases can be systemic, such as systemic lupus erythematosus or localised to particular regions of the body, such as dermatitis.

Cannabinoids have shown to have a variety effects on body systems. Through the CB1 and CB2 receptors, they exert an effect by modulating neurotransmitter and cytokine release. Current research in the role of cannabinoids in the immune system shows that they possess immunosuppressive properties. They can inhibit proliferation of leucocytes, induce apoptosis of T cells and macrophages and reduce secretion of pro-inflammatory cytokines. In mice models, they are effective in reducing inflammation in arthritis, multiple sclerosis, have a positive effect on neuropathic pain and in type 1 diabetes mellitus (Katchan et al Autoimmun. Rev., 2016, 15(6):513-28).

Furthermore, serotonin (5-hydroxytryptamine; 5-HT) is a biogenic amine with a complex and multifaceted biological function. The main receptors and their subtypes are 5-HT1 (5-HT1A, 5-HT1B, 5-HT1D, 5-HT1E and 5-HT1F), 5-HT2 (5-HT2A, 5-HT2B and 5-HT2C), 5-HT3, 5-HT4, 5-HT5 (5-HT5A, 5-HT5B), 5-HT6 and 5-HT7. The receptors are all G-protein coupled receptors (GPCR) except 5-HT3 receptors, which are ionic channels.

Drugs which are capable of either selectively stimulating or inhibiting these receptor subtypes have therapeutic potential in a wide variety of disease conditions. Specifically, agonists of 5-HT receptors are known to be useful in the treatment of autoimmune diseases.

For example, Thompson et. al; Immunology Letters 228 (2020) 45-54; "Psychedelics as a novel approach to treating autoimmune conditions" discloses the use of 5-HT agonists in the treatment of various autoimmune disorders. This is also discussed in Wan et. al: Frontiers in Immunology 11 (2020); "Serotonin: A potent immune cell modulator in autoimmune diseases".

WO 2019/186207 A1 discloses naltrexone and vitamin D for separate, sequential or simultaneous administration, for use in the therapy of an autoimmune disease.

WO 2016/064987 A1 discloses the extraction of pharmaceutically active components from plant materials, and the preparation of a botanical drug substance (BDS) for incorporation into a medicament which may comprise cannabinoids.

Rengasamy et al. (Pharmacol Ther. 2019 Feb;194:107-131) discloses flavonoids as therapeutic agents for autoimmune diseases.

Thompson and Szabo (Immunol Lett. 2020 Dec;228:45-54) discloses various psychedelics for use in treating autoimmune disorders.

As the underlying causes of many autoimmune diseases remain elusive, the development of preventative measures to combat the onset of many autoimmune diseases is unattainable. There is thus a need to develop curative therapies. Moreover, the link between lifestyle factors and the increasing frequency of diagnosis has created an even greater urgency for developing effective treatments that can reverse or bring under control the symptoms of autoimmune diseases.

### Summary of the Invention

The references to methods of treatment in this description are to be interpreted as references to the compounds, pharmaceutical compositions and medicaments of the present invention for use in a method for treatment of the human (or animal) body by therapy.

In a first aspect of the invention, there is provided a pharmaceutical composition comprising naltrexone or a metabolite thereof selected from the list consisting of 6-β-naltrexol, 2-hydroxy-3-methoxy-6β-naltrexol and 2-hydroxy-3 methoxy-naltrexone, or an analogue selected from the group consisting of methylnaltrexone, nalmefene and nalorphine, for use in the treatment of an autoimmune disease within a subject, wherein a therapeutically effective amount of the naltrexone or metabolite thereof or analogue of either is to be administered to the subject in a first treatment phase, wherein after the first treatment phase the subject is to be administered a therapeutically effective amount of a cannabinoid in a second treatment phase, and wherein an agonist of a 5-hydroxytryptamine (5-HT) receptor is to be administered to the subject either sequentially or separately with the naltrexone, the metabolite or analogue thereof, wherein the 5-HT agonist is to be administered subsequent to the naltrexone or metabolite or analogue thereof,
and wherein the composition comprises naltrexone, or the metabolite or analogue thereof, at a dosage amount of between 0.01mg - 10mg.

In a second aspect of the invention, there is provided a pharmaceutical composition comprising a cannabinoid for use in the treatment of an autoimmune disease within a subject, wherein said subject is characterised in having undergone a first treatment phase during which the subject is administered the composition as defined in the first aspect of the invention, and wherein following the first treatment phase a therapeutically amount of said cannabinoid is to be administered to the subject, and wherein an agonist of a 5-hydroxytryptamine (5-HT) receptor is to be administered to the subject either sequentially or separately with the naltrexone, the metabolite or analogue thereof. wherein the 5-HT agonist is to be administered subsequent to the naltrexone or metabolite or analogue thereof.

It has been found by the present inventors that the immunosuppressive properties of cannabinoids is brought about more effectively by combined treatment with naltrexone or a metabolite or analogue thereof. Furthermore, the effectiveness of such treatment is surprisingly dependent on the order in which the two agents are administered, with the most effective regime being a phase of treatment with naltrexone or a metabolite or analogue thereof followed by a phase of treatment with a cannabinoid.

The inventors have also found that treatment with naltrexone or a metabolite or analogue thereof brings about a significant increase in levels of the CB2 receptor in cells, thus allowing the cannabinoid to be more effective.

The inventors of the present invention have discovered that naltrexone, an orally-administered opioid antagonist with the chemical name morphinan-6-one,17-(cyclopropylmethyl)-4,5-epoxy-3,14-dihydroxy-(5α), or a metabolite or analogue thereof, increases serotonin (5-HT) receptor levels and can therefore be used to aid the effectiveness of agonists of 5-HT receptors. This has been demonstrated in the examples of the present application where it can be seen that both higher dose and low dose naltrexone increase the levels of mRNA for one or more of 5-HT receptor proteins. As 5-HT receptor agonists are known to be used in the treatment of autoimmune diseases, this discovery leads to improved efficacy in the treatment of autoimmune diseases with 5-HT receptor agonists.

### Detailed Description of the Invention

The invention provides a specific therapeutic regimen for treating an autoimmune disease in a subject, wherein a cannabinoid is administered after administration of naltrexone or a metabolite thereof or an analogue selected from the group consisting of methylnaltrexone, nalmefene and nalorphine and wherein an agonist of a 5-hydroxytryptamine (5-HT) receptor is to be administered to the subject either sequentially or separately with the naltrexone, the metabolite or analogue, as defined in the claims.

The phased administration of naltrexone or a metabolite or analogue thereof and then a cannabinoid has been found by the inventors to inhibit an autoimmune disease more effectively than separate or simultaneous administration, or phased administration of a cannabinoid and then naltrexone or a metabolite or analogue thereof.

It was also found that levels of the CB2 receptor increased significantly after treatment with naltrexone or a metabolite or analogue thereof. Without wishing to be bound by theory, this increase in CB2 receptor levels, which is the therapeutic target of cannabinoids such as CBD, is feasibly a contributing factor to the increase in the effectiveness of the treatment with CBD.

The inventors of the present invention have discovered that naltrexone, an orally-administered opioid antagonist with the chemical name morphinan-6-one,17-(cyclopropylmethyl)-4,5-epoxy-3,14-dihydroxy-(5α), or a metabolite or analogue thereof, increases serotonin (5-HT) receptor levels and can therefore be used to aid the effectiveness of agonists of 5-HT receptors. This has been demonstrated in the examples of the present application where it can be seen that both higher dose and low dose naltrexone increase the levels of mRNA for one or more of the 5-HT receptor proteins. As 5-HT receptor agonists are known to be used in the treatment of autoimmune diseases, this discovery leads to improved efficacy in the treatment of autoimmune diseases with 5-HT receptor agonists.

In a first aspect of the invention, there is provided a pharmaceutical composition comprising naltrexone or a metabolite thereof selected from the list consisting of 6-β-naltrexol, 2-hydroxy-3-methoxy-6β-naltrexol and 2-hydroxy-3 methoxy-naltrexone, or an analogue selected from the group consisting of methylnaltrexone, nalmefene and nalorphine, for use in the treatment of an autoimmune disease within a subject, wherein a therapeutically effective amount of the naltrexone or metabolite thereof or analogue of either is to be administered to the subject in a first treatment phase, wherein after the first treatment phase the subject is to be administered a therapeutically effective amount of a cannabinoid in a second treatment phase, and wherein an agonist of a 5-hydroxytryptamine (5-HT) receptor is to be administered to the subject either sequentially or separately with the naltrexone, the metabolite or analogue thereof, wherein the 5-HT agonist is to be administered subsequent to the naltrexone or metabolite or analogue thereof,
and wherein the composition comprises naltrexone, or the metabolite or analogue thereof, at a dosage amount of between 0.01mg - 10mg.

In a second aspect of the invention, there is provided a pharmaceutical composition comprising a cannabinoid for use in the treatment of an autoimmune disease within a subject, wherein said subject is characterised in having undergone a first treatment phase during which the subject is administered the composition as defined in first aspect of the invention, and wherein following the first treatment phase a therapeutically amount of said cannabinoid is to be administered to the subject, and wherein an agonist of a 5-hydroxytryptamine (5-HT) receptor is to be administered to the subject either sequentially or separately with the naltrexone, the metabolite or analogue, wherein the 5-HT agonist is to be administered subsequent to the naltrexone or metabolite or analogue thereof.

As used herein, "naltrexone" refers to the compound 17-cyclopropylmethyl-4.5□-epoxy-3,14-dihydroxymorphinan-6-one (with IUPAC name (4R,4aS,7aR,12bS)-3-(cyclopropylmethyl)-4a,9-dihydroxy-2,4,5,6,7a,13-hexahydro-1H-4,12-methanobenzofuro[3,2-e]isoquinoline-7-one) and pharmaceutically acceptable salts, solvates, hydrates, racemates, stereoisomers, clathrates, and polymorphs thereof. Analogues thereof are also envisaged for use as per the invention. Suitable analogues are selected from the group consisting of methylnaltrexone, nalmefene and nalorphine.

The naltrexone is typically in its hydrochloride salt form.

"Low Dose Naltrexone" (LDN) refers to naltrexone administered in "low" doses of less than 0.5 mg/kg, preferably less than 0.2 mg/kg, more preferably between 0.01 mg/kg and 0.08 mg/kg, even more preferably between 0.03 mg/kg and 0.06 mg/kg, most preferably between 0.04 mg/kg and 0.05 mg/kg. Typically, a low dose is up to 4.5 mg per day in total, per patient.

Metabolites of naltrexone are selected from the group consisting of 6-β-naltrexol, 2-hydroxy-3-methoxy-6β-naltrexol and 2-hydroxy-3 methoxy-naltrexone.

The pharmaceutical composition comprises naltrexone or a metabolite or analogue thereof at a dosage amount of between 0.01 mg - 10 mg, preferably 1 mg - 6 mg. In a preferred embodiment, the pharmaceutical composition comprises naltrexone or a metabolite or analogue thereof in an amount of 3 mg to 4.5 mg (i.e. a low dosage). In another preferred embodiment, the pharmaceutical composition comprises naltrexone or a metabolite or analogue thereof in an amount of 5 mg to 10 mg (i.e. a higher dosage amount).

The preferred metabolite of naltrexone is 6-β-naltrexol (6BN), which as used herein refers to the compound N-cyclopropylmethyl-7,8-dihydro-14-hydroxynorisomorphine (with IUPAC name (4R,4aS,7R,7aR,12bS)-3-(cyclopropylmethyl)-1,2,4,5,6,7,7a,13-octahydro-4,12-methanobenzofuro[3,2-e]isoquinoline-4a,7,9-triol), and pharmaceutically acceptable salts, solvates, hydrates racemates, stereoisomers, clathrates, and polymorphs thereof.

The 6BN or analogues etc., may also be administered at "low" or "higher" doses.

In this context, a "low dose" or "higher dose" can be the same as that outlined above for naltrexone.

In certain embodiments, where the agent is 6-β-naltrexol, 6-β-naltrexol is to be administered in an amount effective to increase the blood plasma concentration of 6-β-naltrexol to at least 0.34 ng/ml, preferably at least 3.4 ng/ml, more preferably at least 34 ng/ml, or most preferably at least 340 ng/ml. In certain embodiments, 6-β-naltrexol is to be administered in an amount effective to increase the blood plasma concentration of 6-β-naltrexol to within the range of 0.3 ng/ml to 3,400 ng/ml, preferably to within the range of from 34 ng/ml to 3,400 ng/ml, more preferably within the range of 340 ng/ml to 3,400 ng/ml. The amount effective to achieve such an amount can be determined using any number of conventional techniques known to the person skilled in the art. For example, the skilled person could perform mass spectrometry on a blood plasma sample obtained from the subject in order to determine the increase in the concentration of 6-β-naltrexol within the sample after administration of an amount of 6-β-naltrexol. The effective amount is the amount determined to bring about the desired increase in blood plasma concentration. Typically, the naltrexol will be administered in an amount up to 3 mg per day per patient.

Cannabinoids are a class of compounds understood by the skilled person which comprise those abundantly made by plants of the *Cannabis* genus, as well as endocannabinoids which are synthesised in animals. Synthetic compounds active against CB receptors are also envisaged. When used herein the term can refer to any cannabinoid, but is preferably selected from the list containing cannabidiol, cannabidiolic acid, cannabinol, cannabigerol, cannabivarin, tetrahydrocannabivarin, cannabidivarin, cannabichromene, arachidonoylethanolamine, 2-arachidonoylglycerol, 2-arachidonoyl glyceryl ether, N-arachidonoyl dopamine, virodhamine, dronabinol, nabilone, rimonabant, R-(+)-Met-anandamide, WIN-55,212-2, HU-210, JWH-133, SR141716, SR144528 or combinations thereof, or pharmaceutically acceptable salts, solvates, hydrates, racemates, stereoisomers, clathrates, and polymorphs thereof.

In a preferred embodiment, the cannabinoid is cannabidiol or cannabigerol.

The cannabinoid can be administered at conventional amounts based on the particular cannabinoid and the details of the patient. In certain embodiments, the cannabinoid is to be administered each day in doses of between 10 mg and 1000 mg, preferably between 200 and 800 mg, more preferably between 300 and 500 mg.

In a preferred embodiment, the cannabinoid is selected from the list consisting of cannabidiol, cannabidiolic acid, cannabinol, cannabigerol, cannabivarin, tetrahydrocannabivarin, cannabidivarin, cannabichromene, arachidonoylethanolamine, 2-arachidonoylglycerol, 2-arachidonoyl glyceryl ether, N-arachidonoyl dopamine, virodhamine, dronabinol, nabilone, rimonabant, or combinations thereof.

The method of administration is not particularly limited for the therapeutic agent, but in various embodiments of the invention, naltrexone, a metabolite thereof, or an analogue of either and cannabinoids are administered via the oral, buccal, sublingual, nasal, pulmonary, intravenous, rectal, topical, and transdermal routes. For naltrexone, a metabolite thereof, or an analogue of either, administration is preferably oral. For cannabinoids it is preferably sublingual.

The treatment regimen envisaged comprises a "first treatment phase" and a "second treatment phase". In the first treatment phase, a therapeutically effective amount of naltrexone, a metabolite thereof, or an analogue of either is administered. In the second treatment phase, an effective amount of one or more cannabinoids is administered.

In a preferred embodiment, the first treatment phase is for administration for at least two days.

In a preferred embodiment, the second treatment phase is for administration for at least one day.

It is preferred that the second treatment phase begins only after 1 to 7 days have elapsed from the start of the first treatment phase, more preferably 1 to 4 days, most preferably 1 to 2 days, a "day" being a continuous period of 24 hours.

In a further preferred embodiment of the invention, there is a "recovery phase" in between the end of the first treatment phase and the start of the second treatment phase. The recovery phase is characterised by the absence of administration of either the naltrexone or metabolite or analogue, the 5-HT agonist and the cannabinoid. In one embodiment the recovery phase has a duration of at least one day, or at least two days, preferably in the range of one to seven days, most preferably the duration is two days.

In the present invention, an agonist of a 5-hydroxytryptamine (5-HT) receptor is to be administered to the subject either sequentially or separately with the naltrexone, the metabolite or analogue, wherein the 5-HT agonist is to be administered subsequent to the naltrexone or metabolite or analogue thereof.

As used herein, the term "agonist" has its conventional meaning as used in the art. Agonists of the 5-hydroxytryptamine (5-HT) receptor are compounds that activate one or more of the main receptors or their subtypes: 5-HT1 (5-HT1A, 5-HT1B, 5-HT1D, 5-HT1E and 5-HT1F), 5-HT2 (5-HT2A, 5-HT2B and 5-HT2C), 5-HT3, 5-HT4, 5-HT5 (5-HT5A, 5-HT5B), 5-HT6 and 5-HT7. An agonist of a 5-HT receptor is a full or partial agonist of one or more of the following receptors/receptors subtypes: 5-HT1 (5-HT1A, 5-HT1B, 5-HT1D, 5-HT1E and 5-HT1F), 5-HT2 (5-HT2A, 5-HT2B and 5-HT2C), 5-HT3, 5-HT4, 5-HT5 (5-HT5A, 5-HT5B), 5-HT6 and 5-HT7.

"Simultaneous" administration (outside the claimed invention), as defined herein, includes the administration of the agonist and the naltrexone, the metabolite or analogue within about 2 hours or about 1 hour or less of each other, even more preferably at the same time.

"Separate" administration, as defined herein, includes the administration of the agonist and the naltrexone, the metabolite or analogue, more than about 12 hours, or about 8 hours, or about 6 hours or about 4 hours or about 2 hours apart.

"Sequential" administration, as defined herein, includes the administration of the agonist and the naltrexone, the metabolite or analogue each in multiple aliquots and/or doses and/or on separate occasions. The agonist is administered to the subject after administration of the single unit oral dose pharmaceutical composition.

According to the invention, the naltrexone, the metabolite or analogue is to be administered prior to the administration of the agonist, preferably between 1 to 4 days before administration of the agonist.

In a preferred embodiment, the 5-HT receptor is 5-HT receptor subtype 2A, 2B or 2C. A high density of 5-HT2A receptors is found in many cortical areas. These receptors are particularly concentrated in the frontal cortex. 5-HT2A receptors also are found in high density in the claustrum, a region which is connected to the visual cortex; in parts of the limbic system; and in the basal ganglia and the olfactory nuclei. 5-HT2B receptors are found in the central nervous system, such as the subiculum, the substantia nigra and the peripheral nervous system, such as vascular smooth muscle. 5-HT2C receptors are present in high density in the choroid plexus. It has been proposed that 5-HT-induced activation of 5-HT2C receptors could regulate the composition and volume of the cerebrospinal fluid. 5-HT2C receptors also are found throughout the brain, particularly in areas of the limbic system, including the hypothalamus, hippocampus, septum, neocortex and regions associated with motor behaviour, including the substantia nigra and globus pallidus. Agonists of 5-HT2 receptors are known to be useful in the treatment of autoimmune diseases.

When the agonist is an agonist of 5-HT2 receptors, it is preferably selected from the group consisting of psilocybin, psilocin, mescaline, lysergic acid diethylamide (LSD), ketamine, salvinorin A, ibotenic acid, muscimol, N,N-dimethyltryptamine (DMT), 3,4-methylenedioxymethamphetamine (MDMA), methyldiethanolamine, also known as N-methyl diethanolamine (MDEA), 3,4-methylenedioxy amphetamine (MDA), 4-Bromo-2,5-dimethoxyphenethylamine (2C-B); 1-(8-Bromo-2,3,6,7-tetrahydrobenzo-[1,2-b;4,5-b']difuran-4-yl)2-amino-ethane (2C-B-fly); 4-Ethyl-2,5-dimeth-oxyphenethylamine (2C-E); 4-Ethyl-thio-2,5-dimethoxyphenethylamine (2C-T-2); 4-Ethylthio-2,5-dimethoxy-amphetamine (ALEPH-2); 4-Ethylthio-2,5-dimethoxyphenylbutylamine (4C-T-2); 2,5-Dimethoxy-4-ethoxyamphetamine (MEM); 2,4,5-Trimethox-amphetamine (TMA-2); 3,4,5-Trimethoxamphetamine (TMA); 2,5-Dimethoxy-4-bromo-amphetamine (DOB); 2,5-Dimethoxy-4-iodo-amphetamine (DOI); 2,5-Dimethoxy-4-methylamphetamine (DOM); 2,5-Dimethoxy-4-ethylamphet-amine (DOET); 5-Methoxy-N,N-dimethyl-tryptamine (5-MeO-DMT); N,N-Dipropyltryptamine (DPT); 5-Methoxy-N-methyl-N-isopropyltryptamine (5-MeO-MIPT); N,N-Diisopropyltryptamine (DIPT); 5-Methoxy-N,N-diiso-propyltryptamine (5-MeO-DIPT); 6-Fluoro-N,N-dimethyltryptamine (6-fluoro-DMT); lisuride; ibogaine; cis-2a; RR-2b; SS-2c; 2-Ethyl-5-methoxy-N,N-dimethyltryptamine (EMDT), serotonin hydrochloride, or combinations thereof.

In a preferred embodiment, the 5-HT receptor is 5-HT receptor subtype 3. 5-HT3 receptors are found in high density in peripheral ganglia and nerves, including the superior cervical ganglion and vagus nerve, as well as in the substantia gelatinosa of the spinal cord. The 5-HT3 receptor is homomeric and belongs to the ligand-gated ion channel superfamily. It is a serotonin-gated cation channel that causes the rapid depolarization of neurons. Agonists of 5-HT3 receptors are known to be useful in the treatment of autoimmune diseases.

When the agonist is a 5-HT receptor 3 agonist, preferably it is selected from the group consisting of m-chlorophenylbiguanide hydrochloride, N-methylquipazine dimaleate, PSEM 895, quipazine dimaleate, RS56812 hydrochloride, serotonin hydrochloride, SR7227 hydrochloride, 1-phenylbiguanide hydrochloride, cereulide, 2-methyl-5-HT, alpha-methyltryptamine, bufotenin, chlorophenylbiguanide, ibogaine, varenicline, YM-31636.

In the present invention, the agonist of a 5-HT receptor may be a serotonin analogue. By "serotonin analogue" it is meant a functional analogue of serotonin i.e. compounds showing chemical and biological similarity to serotonin. This may be achieved by making modifications to serotonin in order to prepare a new molecule with similar chemical and biological properties.

In a preferred embodiment, the agonist of a 5-HT receptor is to be administered at its approved therapeutic dose. An approved therapeutic dose will be apparent to one skilled in the art, and may vary according to age, sex, weight, which the skilled person is capable of determining.

The phrases "autoimmune disease" or "autoimmune disorder" are well known in the art, and refer to conditions arising from an abnormal immune response to a normal body part. There are over 80 recognised autoimmune conditions, and the underlying causes of many remain unknown. The development of particular autoimmune disorders is linked to the presence of genetic risk factors, whereas others are associated with exposure to specific environmental or chemical factors. Certain lifestyle factors, such as smoking, exercise, sleep and diet have also been linked to an increased risk of developing certain autoimmune disorders. It is envisaged that the treatment of any autoimmune disorder would benefit from the therapy disclosed herein.

In a particular feature of the invention, the autoimmune disease to be treated is selected from the list consisting of hepatitis, ankylosing spondylitis, Lyme disease, systemic lupus erythematosus, fibromyalgia, myasthenia gravis, Guillain-Barré syndrome, multiple sclerosis, Behçet's disease, chronic fatigue syndrome, Parkinson's, scleroderma, atopic dermatitis, myalgic encephalomyelitis (ME), rheumatoid arthritis, lupus, fibrodysplasia ossificans progressive (FOP), Crohn's disease, auto immune peripheral neuropathy, Hashimoto's disease, Grave's disease, alopecia, PTSD, colitis, eczema, irritable bowel syndrome (IBS) or psoriasis. Preferably, the autoimmune disease is psoriasis.

As used herein, the terms "therapy" and "treating" and "treatment" and "to treat" refer to both 1) therapeutic measures that cure, slow down, and/or halt progression of a diagnosed pathologic condition or disorder and 2) prophylactic or preventative measures that prevent and/or slow the development of a targeted pathologic condition or disorder. Thus, those in need of treatment include those already with the disorder; those prone to have the disorder; and those in whom the disorder is to be prevented. In some instances, a subject is successfully "treated" for an autoimmune disease according to the novel applications of the present invention if the patient shows one or more of the following: for example, a reduction in swelling, pruritus, fatigue, general malaise, fever, skin lesions, hair loss or weight loss. In some instances treatment may result in halting the further progression of symptoms of the disorder or the emergence of symptoms of the disorder.

As used herein, the term "subject" refers to any animal, including but not limited to humans, non-human primates, horses, canines, felines, rodents, and other vertebrates which are to be the recipient of a treatment for cancer. The terms "subject" and "patient" are used interchangeably herein.

In a disclosure, the therapy may also include administering Vitamin D to the subject. The Vitamin D may be administered to the subject before, during or following the treatment regimen. During the treatment regimen the Vitamin D may be administered to the subject at any time, such as during the first treatment phase or during the second treatment phase. Preferably, the administration of Vitamin D in the patient should occur before the disclosed treatment regimen begins.

During the first treatment phase, the Vitamin D may be administered to the patient before and/or after administration of the naltrexone or a metabolite thereof or an analogue thereof. The Vitamin D and the naltrexone or a metabolite thereof or an analogue thereof may be administered simultaneously, sequentially or separately, preferably simultaneously. The Vitamin D may also be continued to be administered to the patient after treatment with the naltrexone ceases, so the Vitamin D is administered in the first and second treatment phases.

During the second treatment phase, the Vitamin D may be administered to the patient before and/or after administration of the cannabinoid. The Vitamin D and the cannabinoid may be administered simultaneously, sequentially or separately, preferably simultaneously. The Vitamin D may also be continued to be administered to the patient after treatment with the cannabinoid ceases, so the Vitamin D is also administered following the treatment regimen.

Preferably during the treatment regimen the Vitamin D should be administered to the subject on a daily basis to maintain the correct Vitamin D levels throughout all phases of treatment.

"Simultaneous" administration, as defined herein, includes the administration of substances within about 2 hours or about 1 hour or less of each other, even more preferably at the same time.

"Separate" administration, as defined herein, includes the administration of substances, more than about 12 hours, or about 8 hours, or about 6 hours or about 4 hours or about 2 hours apart.

"Sequential" administration, as defined herein, includes the administration of substances each in multiple aliquots and/or doses and/or on separate occasions.

As used herein, "vitamin D" refers to vitamin D and any intermediate or product of a metabolic pathway of vitamin D that result in a metabolite that is capable of boosting the cytostatic effect of the active. Metabolite may refer to a vitamin D precursor, which can be incorporated into a vitamin D synthetic pathway occurring naturally within the subject to undergo the therapy of the invention. Alternatively, metabolite may refer to a molecule derived from an anabolic or catabolic process that utilizes vitamin D. Non-limiting examples of vitamin D metabolites include ergocalciferol, cholecalciferol, calcidiol, and calcitriol, 1a-hydroxycholecalciferol, 25-hydroxycholecalciferol, 1a,25-hydroxycholecalciferol, 24,25-hydroxycholecalciferol. An "active" metabolite is a metabolite that can be used in the context of the present invention. Dosage regimes of vitamin D or active metabolites thereof will be well known to the person skilled in the art. The term vitamin D also encompasses pharmaceutically acceptable salts of any of the above. A particularly suitable metabolite of vitamin D for use in the present invention is calcitriol.

In certain disclosures, the vitamin D product is to be administered to the patient in an amount sufficient to bring the subject's blood vitamin D concentration to at least 40 ng/ml, more preferable at least 50 ng/ml. Preferably, the blood vitamin D concentration is raised to within a range of from 40 to 220 ng/ml, more preferably the blood vitamin D concentration is raised to within a range of from 40 to 90 ng/ml.

A sufficient amount can be determined by the skilled person by making a routine assessment of certain parameters of the patient to undergo the administration, such as, but not limited to, age, weight, gender, history of illness and/or other lifestyle factors including smoking, alcohol consumption and the level of exercise. Furthermore the skilled person can ascertain whether a dose has been sufficient to raise the vitamin D blood concentration to a sufficient amount by performing routine biochemical and analytical assays on a biological sample obtained from the subject. Preferably, the sample upon which said analysis is to be performed is blood. Examples of such well known assays include but are not limited to mass spectrometry, where the level of vitamin D or active metabolites thereof can be quantitatively measured. A sufficient amount is therefore an amount that achieves the desired blood vitamin D concentration. The desired concentration can be achieved after single administration or after repeated administrations of a dose of vitamin D or an active metabolite thereof. Where the vitamin D product and the naltrexone product are to be administered simultaneously, it is immaterial whether the vitamin D blood concentration is within the desired range prior to administration of the naltrexone product, provided that the vitamin D product is administered in an amount sufficient to raise the blood vitamin D concentration to within the desired concentration range. Other methods for determining the concentration of vitamin D or active metabolites thereof within a biological sample obtained from the patient will be well known to the skilled person. In certain disclosures, the amount of the vitamin D sufficient to raise the blood vitamin D concentration to beyond a certain level is referred to as the "therapeutically effective amount" of the vitamin D product.

In a disclosure, the Vitamin D administered to the subject is calcitriol. As used herein, "calcitriol" refers to the active metabolite of vitamin D, specifically vitamin D3. Calcitriol is also called 1,25-dihydroxyvitamin-D3 or 1,25-dihydroxycholecalciferol. Its empirical formula is C₂₇H₄₄O₃.

The calcitriol may be administered and absorbed in the oral cavity of the patient. The term oral cavity has its normal meaning in the art and is intended to cover the mouth, the lips, the hard palate, the soft palate, the retromolar trigone, the tongue, gingiva (gums), buccal mucosa (the inner lining of the lips and cheeks), and floor of the mouth under the tongue.

Absorption in the oral cavity can be achieved according to any known methods. Absorption of calcitriol in the oral cavity may be achieved by the sublingual, sublabial, gingival or buccal route. For example, calcitriol can be formulated in an orally disintegrating form that disintegrates and dissolves in the mouth without water before swallowing. It may dissolve in this way within 60 seconds or less, preferably less than 10 seconds.

The calcitriol may be an orally disintegrating tablet or coating, an orodisperse tablet or coating, a mouth-dissolving tablet or coating, a quick-dissolve tablet or coating, a fast-melt tablet or coating, a rapid-disintegrating tablet or coating; or a freeze-dried wafer.

An orally disintegrating tablet/coating is a solid dosage form that contains medicinal substances and disintegrates rapidly (within seconds) without water when placed on the tongue. The drug is released, dissolved, or dispersed in the saliva.

A quick-dissolving tablet/coating (also known as a fast-dissolving, fast-dissolving multiparticulate, rapid-dissolving, mouth-dissolving, fastmelting, or orodispersing tablets) is a solid dosage form that does not require water for swallowing. The tablet dissolves within 60 seconds when placed in the mouth. The active ingredients are absorbed through mucous membranes in the mouth.

A freeze-dried wafer is a quick-dissolving, thin matrix that contains a medicinal agent (in this case calcitriol) that does not need water for swallowing. The wafer disintegrates instantaneously in the oral cavity and releases calcitriol, which dissolves or disperses in the saliva.

This calcitriol may be administered sublingually, sublabially or buccally to the subject by rapidly dissolving in the oral cavity when it comes in contact with saliva. It may be absorbed sublingually.

The calcitriol may be formulated according to known methods such as lyophilisation (freeze-drying), cotton candy process, moulding, sublimation and direct compression. In a disclosure, the calcitriol is formulated as a lyophilised dosage form. Preferably it is a lyophilised liquid solution, suspension or emulsion. This may be achieved according to any known methods for producing dosage forms that dissolve in the oral cavity such as using the following technology: Zydis^{®} , QuickSolv^{®} , Lyoc^{®}, Flashdose^{®} , OraSolve^{®} , Ziplet technology, Frosta^{®} , DuraSolve^{®} , Wowtab^{®}, Durasolv^{®}, Flashtab^{®}, Oraquick^{®}, RapiTab^{®} and Nanomelt^{®} (by Elan).

It has been discovered that the patient absorbing calcitriol in the oral cavity overcomes the problems of variable bioavailability that occurs when calcitriol is swallowed in a tablet. When swallowed, calcitriol has variable bioavailability which means the desired dosage may not be achieved. One way to mitigate this problem is to increase the dose per tablet to give the top-end of the dose. However, by doing this, there would be a danger of causing renal damage from toxicity. It has been discovered that administering calcitriol in the oral cavity allows the desired dosage to be achieved without causing any adverse symptoms associated with renal damage, such as fatigue, loss of appetite or leg swelling. Absorption in the oral cavity also avoids drug metabolism via the stomach and intestines, which means a more efficient delivery of calcitriol.

In one non-claimed aspect of the disclosure, there is provided a method for determining the suitability of a subject, who has an autoimmune disease and has already been treated with naltrexone or a metabolite or analogue thereof, for treatment with a cannabinoid. In this disclosure, a sample is to be obtained from the subject and contacted with a CB2 receptor-specific probe. The concentration of CB2 receptors in the sample is thereby determined and compared with a reference measurement made before the first treatment phase.

As used herein, "suitability" refers to the property of having a high probability of treatment, as defined above, being successful, compared to those subjects deemed unsuitable by the test. A subset of subjects undergoing testing by the method envisaged in one aspect of this disclosure will be deemed to be unsuitable for the second phase of treatment.

The method of determining suitability is intended to be considered by the skilled person alongside other tests known to them and exercising their intuition and judgement.

In certain disclosures, the biological "sample" obtained from the subject for use in the method is blood, plasma, serum, lymph fluid, a tissue, or cells derived from a tissue sample.

As used herein, the "probe" is any moiety which, on contacting the sample obtained from the subject, allows in some way the measurement of the concentration of CB2 receptors in the sample. In one disclosure, this probe is an antibody or other CB2 receptor binding molecule used in the provision of a purer solution of CB2 receptors which can be analysed spectrophotometrically and through calibration using methods known to the skilled person, the concentration of CB2 receptors in the original sample can be determined.

In another aspect of the disclosure it is provided the use of naltrexone, metabolites thereof, or analogue selected from the group consisting of methylnaltrexone, nalmefene and nalorphine, and cannabinoids in the manufacture of a medicament to be administered as part of a "combined treatment regimen", which as used herein refers to the regimen consisting of the first treatment phase, preferable recovery phase, and second treatment phase, as defined above for the treatment of an autoimmune disease.

### Example 1

CD3+ lymphocytes were isolated from the blood of haematologically normal volunteers. Cells placed into 25 cm³ flasks at a concentration of 1x10⁶/ml, and left to equilibrate in an incubator with a humidified atmosphere and 5% CO₂ in air at 37°C. Cells were then treated for 4 h with naltrexone (NTX) (10 uM) or low dose naltrexone (LDN) (10 nM). RNA extraction was performed using Trizol according to standard procedures, before being processed for microarray analysis according to the standard methodologies. Briefly, equal amounts of biotinylated cRNA was hybridised to the Illumina human HT12-v3 arrays for 18 h and subsequently processed according to manufacturer's instructions before scanning on an Illumina BeadArray Reader. The image data were processed using default values in GenomeStudio v2009.1 with imputation of missing data, before loading onto GeneSpring v9.0 for data normalisation and filtering. Data analyses were performed using Excel software, and the ratio of gene expression signals in treated vs. untreated calculated.

The results are shown below in Table 1.

**Table 1**

| Probe ID | Definition | Symbol | UN | NTX | LDN | NTX/ UN | LDN/ UN |
|---|---|---|---|---|---|---|---|
| ILMN_1794095 | 5-HT receptor 1E, mRNA. | HTR1E | 93.05 | 95.70 | 101.20 | 1.03 | 1.09 |
| ILMN_1715496 | 5-HT receptor 2A, mRNA. | HTR2A | 80.00 | 85.30 | 80.00 | 1.07 | 1.00 |
| ILMN_173576 4 | 5-HT receptor 2B, mRNA. | HTR2B | 111.95 | 177.95 | 147.45 | 1.59 | 1.32 |
| ILMN_180135 7 | 5-HT receptor 2C, mRNA. | HTR2C | 80.00 | 89.50 | 85.50 | 1.12 | 1.07 |
| ILMN_166207 0 | 5-HT receptor 3A, transcript variant 2, mRNA. | HTR3A | 80.00 | 80.60 | 82.35 | 1.01 | 1.03 |
| ILMN_168149 2 | 5-HT receptor 3A, transcript variant 1, mRNA. | HTR3A | 85.10 | 86.75 | 94.00 | 1.02 | 1.10 |
| ILMN_237107 9 | 5-HT receptor 3A, transcript variant 2, mRNA. | HTR3A | 100.55 | 151.75 | 124.70 | 1.51 | 1.24 |
| ILMN_167635 0 | 5-HT receptor 4, transcript variant a, mRNA. | HTR4 | 90.35 | 96.10 | 100.20 | 1.06 | 1.11 |
| ILMN_180825 8 | 5-HT receptor 4, transcript variant hb, mRNA. | HTR4 | 80.00 | 88.20 | 93.50 | 1.10 | 1.17 |
| ILMN_174421 7 | 5-HT receptor 7 (adenylate cyclase-coupled), transcript variant a, mRNA. | HTR7 | 80.00 | 86.60 | 80.00 | 1.08 | 1.00 |

As can be seen from the above data, both higher dose and low dose naltrexone increase the levels of mRNA for one or more of 5-HT receptor proteins.

## Claims

1. A pharmaceutical composition comprising naltrexone or a metabolite thereof selected from the list consisting of 6-β-naltrexol, 2-hydroxy-3-methoxy-6β-naltrexol and 2-hydroxy-3 methoxy-naltrexone, or an analogue selected from the group consisting of methylnaltrexone, nalmefene and nalorphine, for use in the treatment of an autoimmune disease within a subject,
wherein a therapeutically effective amount of the naltrexone or metabolite thereof or analogue of either is to be administered to the subject in a first treatment phase,
wherein after the first treatment phase the subject is to be administered a therapeutically effective amount of a cannabinoid in a second treatment phase,
wherein an agonist of a 5-hydroxytryptamine (5-HT) receptor is to be administered to the subject either sequentially or separately with the naltrexone, the metabolite or analogue, wherein the 5-HT agonist is to be administered subsequent to the naltrexone or metabolite or analogue thereof, and
wherein the composition comprises naltrexone, or the metabolite or analogue thereof, at a dosage amount of between 0.01mg - 10mg.

2. A pharmaceutical composition comprising a cannabinoid for use in the treatment of an autoimmune disease within a subject,
wherein said subject is **characterised in** having undergone a first treatment phase during which the subject is administered the composition as defined in claim 1, and
wherein following the first treatment phase a therapeutically amount of said cannabinoid is to be administered to the subject, and
wherein an agonist of a 5-hydroxytryptamine (5-HT) receptor is to be administered to the subject either sequentially or separately with the naltrexone, the metabolite or analogue, wherein the 5-HT agonist is to be administered subsequent to the naltrexone or metabolite or analogue thereof.

3. The pharmaceutical composition for use according to any preceding claim, wherein the first treatment phase is for administration for at least two days, and/or
wherein the second treatment phase is for administration for at least one day.

4. The pharmaceutical composition for use according to any preceding claim, wherein the first treatment phase and second treatment phase are separated by a recovery phase, said recovery phase **characterised by** the absence of administration of either the naltrexone or metabolite or analogue, the 5-HT agonist and the cannabinoid.
preferably wherein the recovery phase is for at least one day, preferably in the range of 1 to 7 days.

5. The pharmaceutical composition for use according to claim 1, wherein the composition comprises naltrexone or 6-β-naltrexol.

6. The pharmaceutical composition for use according to any preceding claim, wherein the cannabinoid is selected from the list consisting of cannabidiol, cannabidiolic acid, cannabinol, cannabigerol, cannabivarin, tetrahydrocannabivarin, cannabidivarin, cannabichromene, arachidonoylethanolamine, 2-arachidonoylglycerol, 2-arachidonoyl glyceryl ether, N-arachidonoyl dopamine, virodhamine, dronabinol, nabilone, rimonabant, or combinations thereof,
preferably wherein the cannabinoid is cannabidiol or cannabigerol.

7. The pharmaceutical composition for use according to any preceding claim, wherein the subject is administered Vitamin D before, during or following the treatment.

8. The pharmaceutical composition for use according to claim 7, wherein the Vitamin D is administered during the first treatment phase and/or wherein the Vitamin D is administered during the second treatment phase.

9. The pharmaceutical composition for use according to claim 7 or claim 8, wherein the Vitamin D is calcitriol,
preferably wherein the calcitriol is administered and absorbed in the oral cavity of the patient.

10. The pharmaceutical composition for use according to any preceding claim, wherein the agonist is an agonist of 5-HT receptor 2 or 3.

11. The pharmaceutical composition for use according to claim 10, wherein the 5-HT agonist is a 5-HT receptor 2 agonist, preferably wherein the agonist is selected from the group consisting of psilocybin, psilocin, mescaline, lysergic acid diethylamide (LSD), ketamine, salvinorin A, ibotenic acid, muscimol, N,N-dimethyltryptamine (DMT), 3,4-methylenedioxymethamphetamine (MDMA), methyldiethanolamine, also known as N-methyl diethanolamine (MDEA), 3,4-methylenedioxy amphetamine (MDA), 4-Bromo-2,5-dimethoxyphenethylamine (2C-B); 1-(8-Bromo-2,3,6,7-tetrahydrobenzo-[1,2-b;4,5-b']difuran-4-yl)2-aminoethane (2C-B-fly); 4-Ethyl-2,5-dimeth-oxyphenethylamine (2C-E); 4-Ethyl-thio-2,5-dimethoxyphenethylamine (2C-T-2); 4-Ethylthio-2,5-dimethoxy-amphetamine (ALEPH-2); 4-Ethylthio-2,5-dimethoxyphenylbutylamine (4C-T-2); 2,5-Dimethoxy-4-ethoxyamphetamine (MEM); 2,4,5-Trimethox-amphetamine (TMA-2); 3,4,5-Trimethoxamphetamine (TMA); 2,5-Dimethoxy-4-bromo-amphetamine (DOB); 2,5-Dimethoxy-4-iodo-amphetamine (DOI); 2,5-Dimethoxy-4-methylamphetamine (DOM); 2,5-Dimethoxy-4-ethylamphet-amine (DOET); 5-Methoxy-N,N-dimethyl-tryptamine (5-MeO-DMT); N,N-Dipropyltryptamine (DPT); 5-Methoxy-N-methyl-N-isopropyltryptamine (5-MeO-MIPT); N,N-Diisopropyltryptamine (DIPT); 5-Methoxy-N,N-diiso-propyltryptamine (5-MeO-DIPT); 6-Fluoro-N,N-dimethyltryptamine (6-fluoro-DMT); lisuride; ibogaine; cis-2a; RR-2b; SS-2c; 2-Ethyl-5-methoxy-N,N-dimethyltryptamine (EMDT), serotonin hydrochloride, and combinations thereof.

12. The pharmaceutical composition for use according to claim 10, wherein the 5-HT agonist is a 5-HT receptor 3 agonist, preferably wherein the agonist is selected from the group consisting of m-chlorophenylbiguanide hydrochloride, N-methylquipazine dimaleate, PSEM 895, quipazine dimaleate, RS56812 hydrochloride, serotonin hydrochloride, SR7227 hydrochloride, 1-phenylbiguanide hydrochloride, cereulide, 2-methyl-5-HT, alpha-methyltryptamine, bufotenin, chlorophenylbiguanide, ibogaine, varenicline, YM-31636.

13. The pharmaceutical composition for use according to claim 1, wherein the composition comprises naltrexone at a dosage amount of between 0.01mg - 10mg, preferably wherein the composition comprises naltrexone at a dosage amount of between 1mg - 6mg, more preferably wherein the composition comprises naltrexone at a dosage amount of between 3mg - 4.5mg.

14. The pharmaceutical composition for use according to claim 2, wherein the subject is **characterised in** having undergone a first treatment phase during which the subject is administered the composition as defined in claim 13.

15. The pharmaceutical composition for use according to any preceding claim, wherein the autoimmune disease is hepatitis, ankylosing spondylitis, Lyme disease, systemic lupus erythematosus, fibromyalgia, myasthenia gravis, Guillain-Barré syndrome, multiple sclerosis, Behçet's disease, chronic fatigue syndrome, Parkinson's, scleroderma, atopic dermatitis, myalgic encephalomyelitis (ME), rheumatoid arthritis, lupus, fibrodysplasia ossificans progressive (FOP), Crohn's disease, auto immune peripheral neuropathy, Hashimoto's disease, Grave's disease, alopecia, colitis, eczema, irritable bowel syndrome (IBS) or psoriasis.

## Patentansprüche

1. Pharmazeutische Zusammensetzung, umfassend ein Naltrexon oder einen Metaboliten davon, ausgewählt aus der Liste, bestehend aus 6-β-Naltrexol, 2-Hydroxy-3-methoxy-6β-naltrexol und 2-Hydroxy-3-methoxy-naltrexon, oder einem Analogon, ausgewählt aus der Gruppe, bestehend aus Methylnaltrexon, Nalmefen und Nalorphin, zur Verwendung bei der Behandlung einer Autoimmunkrankheit bei einer Person,
wobei der Person eine therapeutisch wirksame Menge des Naltrexons oder Metaboliten davon oder Analogons von einem davon in einer ersten Behandlungsphase zu verabreichen ist,
wobei der Person nach der ersten Behandlungsphase eine therapeutisch wirksame Menge eines Cannabinoids in einer zweiten Behandlungsphase zu verabreichen ist,
wobei der Person ein Agonist eines 5-Hydroxytryptamin- (5-HT-) Rezeptors entweder im Anschluss an oder separat mit dem Naltrexon, dem Metaboliten oder dem Analogon zu verabreichen ist, wobei der 5-HT-Agonist nach dem Naltrexon oder Metaboliten oder Analogon davon zu verabreichen ist, und
wobei die Zusammensetzung Naltrexon oder den Metaboliten oder das Analogon davon in einer Dosierungsmenge zwischen 0,01 mg - 10 mg umfasst.

2. Pharmazeutische Zusammensetzung, umfassend ein Cannabinoid zur Verwendung bei der Behandlung einer Autoimmunkrankheit einer Person,
wobei die Person **dadurch gekennzeichnet ist, dass** sie einer ersten Behandlungsphase unterzogen wurde, während der der Person die Zusammensetzung wie in Anspruch 1 definiert verabreicht wurde, und
wobei der Person im Anschluss an die erste Behandlungsphase eine therapeutisch wirksame Menge des Cannabinoids zu verabreichen ist, und
wobei der Person ein Agonist eines 5-Hydroxytryptamin- (5-HT-) Rezeptors entweder im Anschluss an oder separat mit dem Naltrexon, dem Metaboliten oder dem Analogon zu verabreichen ist, wobei der 5-HT-Agonist nach dem Naltrexon oder Metaboliten oder Analogon davon zu verabreichen ist.

3. Pharmazeutische Zusammensetzung zur Verwendung nach einem vorstehenden Anspruch, wobei die erste Behandlungsphase zur Verabreichung mindestens zwei Tage dauert, und/oder
wobei die zweite Behandlungsphase zur Verabreichung mindestens einen Tag dauert.

4. Pharmazeutische Zusammensetzung zur Verwendung nach einem vorstehenden Anspruch, wobei die erste Behandlungsphase und die zweite Behandlungsphase durch eine Erholungsphase getrennt sind, wobei die Erholungsphase durch die Abwesenheit einer Verabreichung entweder des Naltrexon oder des Metaboliten oder Analogons, des 5-HT-Agonisten und des Cannabinoids gekennzeichnet ist,
wobei die Erholungsphase vorzugsweise mindestens einen Tag, vorzugsweise im Bereich von 1 bis 7 Tagen dauert.

5. Pharmazeutische Zusammensetzung zur Verwendung nach Anspruch 1, wobei die Zusammensetzung Naltrexon oder 6-β-Naltrexol umfasst.

6. Pharmazeutische Zusammensetzung zur Verwendung nach einem vorstehenden Anspruch, wobei das Cannabinoid aus der Liste ausgewählt ist, bestehend aus Cannabidiol, Cannabidiolsäure, Cannabinol, Cannabigerol, Cannabivarin, Tetrahydrocannabivarin, Cannabidivarin, Cannabichromen, Arachidonoylethanolamin, 2-Arachidonoylglycerol, 2-Arachidonoylglycerylether, N-Arachidonoyldopamin, Virodhamin, Dronabinol, Nabilon, Rimonaban oder Kombinationen davon,
wobei das Cannabinoid vorzugsweise Cannabidiol oder Cannabigerol ist.

7. Pharmazeutische Zusammensetzung zur Verwendung nach einem vorstehenden Anspruch, wobei der Person vor, während oder nach der Behandlung Vitamin D verabreicht wird.

8. Pharmazeutische Zusammensetzung zur Verwendung nach Anspruch 7, wobei das Vitamin D während der ersten Behandlungsphase verabreicht wird und/oder wobei das Vitamin D während der zweiten Behandlungsphase verabreicht wird.

9. Pharmazeutische Zusammensetzung zur Verwendung nach Anspruch 7 oder 8, wobei das Vitamin D Calcitriol ist,
wobei das Calcitriol vorzugsweise in der Mundhöhle des Patienten verabreicht und absorbiert wird.

10. Pharmazeutische Zusammensetzung zur Verwendung nach einem vorstehenden Anspruch, wobei der Agonist ein Agonist von 5-HT-Rezeptor-2 oder -3 ist.

11. Pharmazeutische Zusammensetzung zur Verwendung nach Anspruch 10, wobei der 5-HT-Agonist ein 5-HT-Rezeptor-2-Agonist ist, wobei der Agonist vorzugsweise aus der Gruppe ausgewählt ist, bestehend aus Psilocybin, Psilocin, Mescalin, Lysergsäurediethylamid (LSD), Ketamin, Salvinorin A, Ibotensäure, Muscimol, N,N-Dimethyltryptamin (DMT), 3,4-Methylenedioxymethamphetamin (MDMA), Methyldiethanolamin, auch bekannt als N-methyl-diethanolamin (MDEA), 3,4-Methylenedioxy-amphetamin (MDA), 4-Bromo-2,5-dimethoxyphenethylamin (2C-B); 1-(8-Bromo-2,3,6,7-tetrahydrobenzo-[1,2-b;4,5-b']difuran-4-yl)2-amino-ethan (2C-B-fly); 4-Ethyl-2,5-dimeth-oxyphenethylamin (2C-E); 4-Ethyl-thio-2,5-dimethoxyphenethylamin (2C-T-2); 4-Ethylthio-2,5-dimethoxy-amphetamin (ALEPH-2); 4-Ethylthio-2,5-dimethoxyphenylbutylamin (4C-T-2); 2,5-Dimethoxy-4-ethoxyamphetamin (MEM); 2,4,5-Trimethox-amphetamin (TMA-2); 3,4,5-Trimethoxamphetamin (TMA); 2,5-Dimethoxy-4-bromo-amphetamin (DOB); 2,5-Dimethoxy-4-iodo-amphetamin (DOI); 2,5-Dimethoxy-4-methylamphetamin (DOM); 2,5-Dimethoxy-4-ethylamphetamin (DOET); 5-Methoxy-N,N-dimethyltryptamin (5-MeO-DMT); N,N-Dipropyltryptamin (DPT); 5-Methoxy-N-methyl-N-isopropyltryptamin (5-MeO-MIPT); N,N-Diisopropyltryptamin (DIPT); 5-Methoxy-N,N-diisopropyltryptamin (5-MeO-DIPT); 6-Fluoro-N,N-dimethyltryptamine (6-fluoro-DMT); Lisurid; Ibogain; cis-2a; RR-2b; SS-2c; 2-Ethyl-5-methoxy-N,N-dimethyltryptamin (EMDT), Serotoninhydrochlorid und Kombinationen davon.

12. Pharmazeutische Zusammensetzung zur Verwendung nach Anspruch 10, wobei der 5-HT-Agonist ein 5-HT-Rezeptor-3-Agonist ist, wobei der Agonist vorzugsweise aus der Gruppe ausgewählt ist, bestehend aus m-Chlorphenylbiguanidhydrochlorid, N-Methylquipazindimaleat, PSEM 895, Quipazindimaleat, RS56812 Hydrochlorid, Serotoninhydrochlorid, SR7227 Hydrochlorid, 1-Phenylbiguanidhydrochlorid, Cereulid, 2-Methyl-5-HT, alpha-Methyltryptamin, Bufotenin, Chlorphenylbiguanid, Ibogain, Vareniclin, YM-31636.

13. Pharmazeutische Zusammensetzung zur Verwendung nach Anspruch 1, wobei die Zusammensetzung Naltrexon in einer Dosierungsmenge zwischen 0.01 mg - 10 mg umfasst, wobei die Zusammensetzung Naltrexon vorzugsweise in einer Dosierungsmenge zwischen 1 mg - 6 mg umfasst, wobei die Zusammensetzung Naltrexon bevorzugter in einer Dosierungsmenge zwischen 3 mg - 4,5 mg umfasst.

14. Pharmazeutische Zusammensetzung zur Verwendung nach Anspruch 2, wobei die Person **dadurch gekennzeichnet ist, dass** sie einer ersten Behandlungsphase unterzogen wurde, während der der Person die Zusammensetzung wie in Anspruch 13 definiert verabreicht wurde.

15. Pharmazeutische Zusammensetzung zur Verwendung nach einem vorstehenden Anspruch, wobei es sich bei der Autoimmunkrankheit um Hepatitis, Spondylitis ankylosans, Lyme-Borreliose, systemischer Lupus erythematodes, Fibromyalgie, Myasthenia gravis, Guillain-Barré-Syndrom, Multiple Sklerose, Behçet-Syndrom, chronisches Erschöpfungssyndrom, Parkinson, Sklerodermie, atopische Dermatitis, myalgische Enzephalomyelitis (ME), rheumatoide Arthritis, Lupus, Fibrodysplasia ossificans progressiva (FOP), Morbus Crohn, autoimmune periphere Neuropathie, Hashimoto-Thyreoiditis, Morbus Basedow, Alopezie, Colitis, Ekzem, Reizdarmsyndrom (IBS) oder Psoriasis handelt.

## Revendications

1. Composition pharmaceutique comprenant de la naltrexone ou un métabolite de celle-ci choisi dans la liste consistant en le 6-β-naltrexol, le 2-hydroxy-3-méthoxy-6β-naltrexol et la 2-hydroxy-3 méthoxy-naltrexone, ou un analogue choisi dans le groupe consistant en la méthylnaltrexone, le nalméfène et la nalorphine, pour une utilisation dans le traitement d'une maladie auto-immune chez un sujet,
dans laquelle une quantité thérapeutiquement efficace de la naltrexone ou d'un métabolite de celle-ci ou d'un analogue de l'un ou l'autre doit être administrée au sujet dans une première phase de traitement,
dans laquelle, après la première phase de traitement, une quantité thérapeutiquement efficace d'un cannabinoïde doit être administrée au sujet dans une seconde phase de traitement,
dans laquelle un agoniste d'un récepteur de 5-hydroxytryptamine (5-HT) doit être administré au sujet soit séquentiellement, soit séparément avec la naltrexone, le métabolite ou l'analogue, dans laquelle l'agoniste 5-HT doit être administré après la naltrexone ou le métabolite ou l'analogue de celle-ci, et
dans laquelle la composition comprend de la naltrexone, ou le métabolite ou l'analogue de celle-ci, à une quantité de dosage entre 0,01 mg et 10 mg.

2. Composition pharmaceutique comprenant un cannabinoïde pour une utilisation dans le traitement d'une maladie auto-immune chez un sujet,
dans laquelle ledit sujet est **caractérisé en ce qu'**il a subi une première phase de traitement au cours de laquelle la composition telle que définie dans la revendication 1 est administrée au sujet, et
dans laquelle, à la suite de la première phase de traitement, une quantité thérapeutique dudit cannabinoïde doit être administrée au sujet, et
dans laquelle un agoniste d'un récepteur de 5-hydroxytryptamine (5-HT) doit être administré au sujet soit séquentiellement, soit séparément avec la naltrexone, le métabolite ou l'analogue, dans laquelle l'agoniste 5-HT doit être administré après la naltrexone ou le métabolite ou l'analogue de celle-ci.

3. Composition pharmaceutique pour une utilisation selon une quelconque revendication précédente, dans laquelle la première phase de traitement doit être administrée pendant au moins deux jours, et/ou
dans laquelle la seconde phase de traitement doit être administrée pendant au moins un jour.

4. Composition pharmaceutique pour une utilisation selon une quelconque revendication précédente, dans laquelle la première phase de traitement et la seconde phase de traitement sont séparées par une phase de récupération, ladite phase de récupération étant **caractérisée par** l'absence d'administration de la naltrexone ou du métabolite ou de l'analogue, de l'agoniste 5-HT et du cannabinoïde,
de préférence dans laquelle la phase de récupération dure au moins un jour, de préférence dans la plage de 1 à 7 jours.

5. Composition pharmaceutique pour une utilisation selon la revendication 1, dans laquelle la composition comprend de la naltrexone ou du 6-β-naltrexol.

6. Composition pharmaceutique pour une utilisation selon une quelconque revendication précédente, dans laquelle le cannabinoïde est choisi dans la liste consistant en le cannabidiol, l'acide cannabidiolique, le cannabinol, le cannabigérol, la cannabivarine, la tétrahydrocannabivarine, la cannabidivarine, le cannabichromène, l'arachidonoyléthanolamine, le 2-arachidonoylglycérol, le 2-arachidonoylglycéryléther, la *N-*arachidonoyldopamine, la virodhamine, le dronabinol, la nabilone, le rimonabant, ou des combinaisons de ceux-ci,
de préférence dans laquelle le cannabinoïde est le cannabidiol ou le cannabigérol.

7. Composition pharmaceutique pour une utilisation selon une quelconque revendication précédente, dans laquelle de la vitamine D est administrée au sujet avant, pendant ou après le traitement.

8. Composition pharmaceutique pour une utilisation selon la revendication 7, dans laquelle la vitamine D est administrée pendant la première phase de traitement et/ou dans laquelle la vitamine D est administrée pendant la seconde phase de traitement.

9. Composition pharmaceutique pour une utilisation selon la revendication 7 ou la revendication 8, dans laquelle la vitamine D est le calcitriol,
de préférence dans laquelle le calcitriol est administré et absorbé dans la cavité buccale du patient.

10. Composition pharmaceutique pour une utilisation selon une quelconque revendication précédente, dans laquelle l'agoniste est un agoniste du récepteur 5-HT 2 ou 3.

11. Composition pharmaceutique pour une utilisation selon la revendication 10, dans laquelle l'agoniste 5-HT est un agoniste du récepteur 5-HT 2, de préférence dans laquelle l'agoniste est choisi dans le groupe consistant en la psilocybine, la psilocine, la mescaline, le diéthylamide de l'acide lysergique (LSD), la kétamine, la salvinorine A, l'acide iboténique, le muscimol, la N,N-diméthyltryptamine (DMT), la 3,4-méthylènedioxyméthamphétamine (MDMA), la méthyldiéthanolamine, également connue sous le nom de N-méthyldiéthanolamine (MDEA), la 3,4-méthylènedioxyamphétamine (MDA), la 4-bromo-2,5-diméthoxyphénéthylamine (2C-B) ; le 1-(8-bromo-2,3,6,7-tétrahydrobenzo-[1,2-b;4,5-b']difuran-4-yl)-2-amino-éthane (2C-B-fly) ; la 4-éthyl-2,5-diméthoxyphénéthylamine (2CE) ; la 4-éthylthio-2,5-diméthoxyphénéthylamine (2C-T-2) ; la 4-éthylthio-2,5-diméthoxyamphétamine (ALEPH-2) ; la 4-éthylthio-2,5-diméthoxyphénylbutylamine (4C-T-2) ; la 2,5-diméthoxy-4-éthoxyamphétamine (MEM) ; la 2,4,5-triméthoxamphétamine (TMA-2) ; la 3,4,5-triméthoxamphétamine (TMA) ; la 2,5-diméthoxy-4-bromoamphétamine (DOB) ; la 2,5-diméthoxy-4-iodoamphétamine (DOI) ; la 2,5-diméthoxy-4-méthylamphétamine (DOM); la 2,5-diméthoxy-4-éthylamphétamine (DOET) ; la 5-méthoxy-N,N-diméthyltryptamine (5-MeO-DMT) ; la N,N-dipropyltryptamine (DPT) ; la 5-méthoxy-N-méthyl-N-isopropyltryptamine (5-MeO-MIPT) ; la N,N-diisopropyltryptamine (DIPT) ; la 5-méthoxy-N,N-diisopropyltryptamine (5-MeO-DIPT) ; la 6-fluoro-N,N-diméthyltryptamine (6-fluoro-DMT) ; le lisuride ; l'ibogaïne ; cis-2a ; RR-2b ; SS-2c; la 2-éthyl-5-méthoxy-N,N-diméthyltryptamine (EMDT), le chlorhydrate de sérotonine, et des combinaisons de ceux-ci.

12. Composition pharmaceutique pour une utilisation selon la revendication 10, dans laquelle l'agoniste 5-HT est un agoniste du récepteur 5-HT 3, de préférence dans laquelle l'agoniste est choisi dans le groupe consistant en le chlorhydrate de m-chlorophénylbiguanide, le dimaléate de N-méthylquipazine, le PSEM 895, le dimaléate de quipazine, le chlorhydrate de RS56812, le chlorhydrate de sérotonine, le chlorhydrate de SR7227, le chlorhydrate de 1-phénylbiguanide, la céréulide, la 2-méthyl-5-HT, l'alpha-methyltryptamine, la bufoténine, le chlorophénylbiguanide, l'ibogaïne, la varénicline et le YM-31636.

13. Composition pharmaceutique pour une utilisation selon la revendication 1, dans laquelle la composition comprend de la naltrexone à une quantité de dosage entre 0,01 mg et 10 mg, de préférence dans laquelle la composition comprend de la naltrexone à une quantité de dosage entre 1 mg et 6 mg, plus préférentiellement dans laquelle la composition comprend de la naltrexone à une quantité de dosage entre 3 mg et 4,5 mg.

14. Composition pharmaceutique pour une utilisation selon la revendication 2, dans laquelle le sujet est **caractérisé en ce qu'**il a subi une première phase de traitement au cours de laquelle la composition telle que définie dans la revendication 13 est administrée au sujet.

15. Composition pharmaceutique pour une utilisation selon une quelconque revendication précédente, dans laquelle la maladie auto-immune est l'hépatite, la spondylarthrite ankylosante, la maladie de Lyme, le lupus érythémateux disséminé, la fibromyalgie, la myasthénie grave, le syndrome de Guillain-Barré, la sclérose en plaques, la maladie de Behçet, le syndrome de fatigue chronique, la maladie de Parkinson, la sclérodermie, la dermatite atopique, l'encéphalomyélite myalgique (EM), la polyarthrite rhumatoïde, le lupus, la fibrodysplasie ossificante progressive (FOP), la maladie de Crohn, la neuropathie périphérique auto-immune, la maladie d'Hashimoto, la maladie de Graves, l'alopécie, la colite, l'eczéma, le syndrome de l'intestin irritable (SII) ou le psoriasis.
